# EUROPEAN PATENT APPLICATION

(11) **EP 1 795 895 A1**
(43) Date of publication of application: **13.06.2007**
(21) Application number: 05026845.7
(22) Date of filing: 08.12.2005
(51) Int. Cl.: G01N 33/50

(54) **A tissue-based assay system for Alzheimer-specific degeneration and pathology**

(71) Applicant: KeyNeurotek AG, 39120 Magdeburg (DE)
(72) Inventor: Mack, Till, Dr., 39104 Magdeburg (DE); Striggow, Frank, Dr., 39175 Gerwisch (DE); Hinners, Ina, Dr., 39104 Magdeburg (DE)
(74) Representative: Kalhammer, Georg

(57) **Abstract**

The present invention relates to modified brain slice cultures and assay systems based thereon. In particular, the invention relates to a tissue-based assay system for screening agents capable of modulating etiopathology of neuronal cells, in particular in the context of Alzheimer's disease related brain lesions.

## Description

The present invention relates to modified brain slice cultures and assay systems based thereon. In particular, the invention relates to a tissue-based assay system for screening agents capable of modulating etiopathology of neuronal cells, in particular in the context of Alzheimer's disease related brain lesions.

The hallmarks of Alzheimer's disease (AD), one of the most serious health problems in the industrialised world, are brain lesions called senile plaques and neurofibrillary tangles (NFT), formed from different proteins, that are associated with nerve cells not communicating with each other and eventually dying. The result is a progressive deterioration of memory, learning and language. Since the molecular characterisations of these lesions, there has been controversy over how these lesions and their constituent molecules are pathogenically related to each other and to the neuronal and synaptic losses that characterise AD. A key part of this debate has been the observation that the pathogenic mutations that underlie the autosomal dominant forms of the disease - mutations in APP or the presenilins - lead to increased production of the Aβ42 peptide in tissues from affected individuals. Consequently, transgenic mouse models for AD overexpressing mutant APP alone or with mutant presenilin have been generated. However, despite developing senile plaques, these animals lack NFTs and, what is more, exhibit very little neuronal loss (Higgins & Jacobsen, 2003). This has limited their use as models of AD and called for models that independently develop AD-like tau pathology and, most importantly, do exhibit substantial neuronal loss.

Model systems that recapitulate pathological mechanisms of a particular disease make powerful tools for drug development. Although rodents do not develop Alzheimer's disease, underlying mechanisms of neuron degeneration have been approximated in animal models via introduction of neurodegeneration-linked human genes. Intraneuronal depositions of microtubule-associated protein tau in filamentous aggregates constitute a pathological hallmark of neurofibrillary degeneration that is characteristic of Alzheimer's disease (AD) and related disorders known collectively as tauopathies. Some animal models may reflect pathomechanisms in AD quite closely since hallmark fibril inclusions develop in AD-relevant brain regions, i.e. the limbic system including the hippocampus, in a stereotypic manner as has been described for Alzheimer patients. Provoking tau-linked degeneration in Alzheimer-typical brain regions like the hippocampus can thus be regarded as relevant Alzheimer etiopathology model system *in vivo*.

Neurofibrillary tangles (NFTs) are the most common intraneuronal inclusions in the brain of patients with neurodegenerative diseases and have been implicated in mediating neuronal death and cognitive deficits. Formation of such fibril inclusions, consisting of hyperphosphorylated tau in multiple isoforms, correlates with cognitive decline in AD. Mutations in the gene encoding tau protein cause frontotemporal dementia and parkinsonism linked to chromosome 17 (FTDP-17), thereby proving that tau dysfunction can directly result in neurodegeneration. Expression of human tau containing the most common FTDP-17 mutation (P301 L) results in motor and behavioural deficits in transgenic mice, with age- and gene-dose-dependent development of NFTs.

Up until now, the need for an assay that allows for rapid drug screening and faithfully recapitulates tau-linked neurodegeneration in the hippocampus has not been met. Less specific and therefore less significant cell culture models have been developed expressing tau variants in cells. WO 2004/017072 A1 discloses a cell culture system for screening and testing of modulating agents of neurodegenerative diseases associated with the formation of neurofibrillary tangles, in particular Alzheimer's disease. Ko et al. (2004) describe studies on assembly of tau aggregates in human neuroblastoma cells. Although tau aggregates resembling neurofibrillar pathology have been observed, significant differences between adult hippocampal neurons and neuronal cells lines of neuroblastoma origin may cast scepticism on the predictive value of these models. In addition, recent studies in transgenic mice surprisingly demonstrated that progressive accumulation of NFTs was not sufficient to cause cognitive decline or neuronal death in a tauophathy model (SantaCruz et al., 2005).

One problem of the present invention is to provide a cell system for drug screening which faithfully recapitulates tau-linked neurodegeneration. The cells in the system should show hallmarks of Alzheimer's disease similar to degenerating neural cells from suffering patients.

It has surprisingly been found that transient expression of human tau_{P301L} in organotypic hippocampal slice cultures from rat brain causes degeneration of dendrites and axons. The transduced brain slices recapitulate tau-linked neurodegeneration and are therefore useful as an *ex vivo* cell system for rapid drug screening. In addition, it has been unexpectedly observed that expression of this tau mutant causes progressive abnormal phosphorylation of tau at Serine 202 and Threonine 205, which precedes degeneration and may therefore be of prime diagnostic value as has been described in Alzheimer patients (Braak & Braak, 1995). These findings emphasise the importance of aberrant phosphoepitopes including phosphorylated serine 202/threonine 205 (i.e. the AT8 epitope) as diagnostic markers for early degenerative aberrations preceding degeneration rather than resulting from it. A disease-like correlation between diagnostic phosphoepitopes and axon or dendrite degeneration has not been established in cell culture models as yet.

The present invention therefore relates to a method for preparing a modified brain slice culture, comprising transfecting or transducing at least one brain slice with a recombinant vector which comprises a polynucleotide encoding tau protein or a variant thereof.

Another aspect of the invention is a method for preparing a test system which recapitulates tau-linked neurodegeneration, comprising transfecting or transducing at least one brain slice with a recombinant vector which comprises a polynucleotide encoding tau protein or an isoform thereof. The test system is useful in drug screening.

Another aspect of the invention is a method for preparing a cell system, comprising transfecting or transducing at least one brain slice with a recombinant vector which comprises a polynucleotide encoding tau protein or an isoform thereof. The cell system preferably recapitulates tau-linked neurodegeneration. The cell system may be used for drug screening.

### Brain slice cultures

As used herein, the term "brain slice culture" means "organotypic brain slice culture" and refers to sections or explants of brain tissue which are maintained in culture. A skilled artisan can readily employ art known organotypic brain slice culture methods for use in the present invention. Organotypic brain slice culture can employ sections of whole brain tissue or explants obtained from specific regions of the brain. Any region can be used to generate an organotypic brain slice culture. However, the preferred source of the organotypic brain slice culture is explants obtained from specific regions of the brain, preferably the hippocampus region. Most preferably, the brain slice contains pyramidal neurons.

Any mammal can be used as a tissue source for the explant that is used to generate the organotypic brain slice culture used in the present method so long as the animal can serve as a tissue source and the organotypic slice culture can be established and maintained for a period sufficient to conduct the present methods. Such mammals include, but are not limited to rats, mice, guinea pigs, monkeys and rabbits. Usually, the mammal is a non-human mammal. The method of the invention may further comprise the step of obtaining a brain slice from the mammal, or providing a brain slice culture. The method may further comprise the step of culturing or cultivating the brain slice prior to transduction or transfection.

The mammal used as a tissue source can be a wild-type mammal or can be a mammal that has been altered genetically to contain and express an introduced gene. For example, the animal may be a transgenic animal, such as a transgenic mouse, that has been altered to express neural production of the β-amyloid precursor protein (Quon et al. (1991) Nature 35:598-607; Higgins et al. (1995) Proc Natl Acad Sci USA 92:4402-4406). In that embodiment, the animal will preferably be altered to express a β-amyloid precursor protein that is derived or based on human β-amyloid sequences. In one embodiment, the mammal used as a tissue source is not a transgenic mammal that has been altered genetically to express tau protein or a variant thereof.

The mammal used as a tissue source can be of any age. In one embodiment, the mammalian tissue source will be a neonatal mammal. The mammal used as tissue source may have an age of about 1 to about 20 days, preferably of about 3 to about 15 days, more preferably of about 5 to about 12 days, still more preferably of about 7 to about 10 days, most preferably of about 8 to about 9 days.

To obtain tissues for culturing from live animals, the animal is preferably quickly killed and decapitated, this generally being performed simultaneously. The brain is then rapidly removed to a dissection media buffered to physiological pH. An example of such a media is a minimal essential media (MEM) buffered with 10 mM Tris, pH 7.2, and supplemented with antibiotic.

The brain or desired brain region is then isolated under aseptic conditions. Entire brain tissue can be used to establish an organotypic brain slice culture. Alternatively, a specific area or region of the brain can be used as an explant source. The preferred regions for the source of the organotypic brain slice culture are the hippocampus and cortex.

Next small regions are separated from the tissue as slices or explants such that the surface to volume ratio allows exchange between the center of the tissue and the media. A variety of procedures can be employed to section or divide the brain tissues. For example, sectioning devices can be employed. The size/thickness of the tissue section will be based primarily on the tissue source and the method used for sectioning/division. For example, preferred segments are from about 300 to about 600 µm, preferably from about 300 to about 500 µm, most preferably from about 350 µm to about 450 µm in diameter and are made using a tissue chopper, razor blade, or other appropriate sectioning/microtome blade.

After sectioning, sections are separated and damaged tissue removed. The sections of brain tissue are preferably manipulated in dissecting media and placed on culture plate inserts in culture media. Excess media is drawn off, and the culture is placed in an incubator.

The choice of culture media and culture conditions depends on the intended use, the source of tissue, and the length of time before the section is used in the present method. Examples of culture media include, but is not limited to 25% horse serum, 50% minimum essential media, 25% Hank's media, supplemented with antibiotic and L-glutamine. Examples of culture condition include, but are not limited to, 37° C, 5% CO₂.

Cultures can be maintained for up to 8 weeks, under ideal conditions. However, organotypic brain slice cultures are preferably used after they have stabilized following the trauma of transfer to culture, but before onset of decline. In general, it is preferable to use the slice cultures from about 1 week to about 4 weeks after they have been generated.

### Tau protein

The tau protein is preferably human tau protein. The amino acid sequence of wild type human tau protein is shown in SEQ ID NO:1. (Homo sapiens microtubule-associated protein tau (MAPT): NM 016834/NP_058518). The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" includes any shorter or longer version of a polypeptide or protein. Variants comprise proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The terms "variant" and "isoform" are used interchangeably herein. In one embodiment the tau protein or variant thereof according to the present application is capable of causing degeneration of dendrites and/or axons upon transduction of brain slices with a vector comprising DNA encoding said tau protein.

Various isoforms of tau protein have been described. Known tau isoforms are summarized in Mandelkow & Mandelkow (1998) or Sergeant et al. (2005) Biochimica et Biophysica Acta 1739:179-197. The amino acid sequences of these tau variants/mutants and the nucleotide sequences encoding them are incorporated herein by reference. Preferred tau variants in accordance with this invention are tau mutants causing frontotemporal dementia and parkinsonism linked to chromosome 17 (FTDP-17). The most preferred tau variant in accordance with this invention has the mutation P301L which has been described to result in motor and behavioural deficits in transgenic mice, with age- and gene-dose-dependent development of NFTs. The amino acid sequence of the 0N4R isoform of human tau harbouring the P301 L mutation comprises the amino acid sequence as shown in SEQ ID NO:3 (amino acid sequence P301L mutant: 275 VQIINKKLDLSNVQSKCGSKDNIKHV**L**GGGS 305). The numbering of amino acids in the human tau sequences as used herein refers to the tau isoform having 441 amino acids which is shown in SEQ ID NO:2.

cDNA sequences encoding tau proteins are known in the art (e.g. Gen-ID NM016834). The skilled person can therefore easily manipulate the DNA by known techniques to provide polynucleotides that encode the desired tau protein or variant thereof.

### Transfection and transduction

As used herein, the term "transduction," is used to describe the delivery and introduction of polynucleotide to eukaryotic cells using viral mediated delivery systems, such as, adenoviral, AAV, retroviral, or plasmid delivery gene transfer methods. These methods are known to those of skill in the art, with the exact compositions and execution being apparent in light of the present disclosure.

As used herein, the term "transfection" is used to describe the delivery and introduction of polynucleotide to a cell using non-viral mediated means, these methods include, e.g., calcium phosphate- or dextran sulfate-mediated transfection; electroporation; glass projectile targeting; and the like. These methods are known to those of skill in the art, with the exact compositions and execution being apparent in light of the present disclosure.

Preferably the transfection or transduction is transient. This generally refers to transient expression of the DNA construct introduced into the cells. The expression of the tau protein or the variant thereof usually peaks at around day 7-8 post transfection or transduction.

Transfection or transduction is preferably performed about 3 days to about 10 days, preferably about 4 days to about 6 days after the brain slice culture has been prepared.

A "vector" is a replicon, such as plasmid, phage, cosmid, or virus to which another polynucleotide segment may be operably inserted so as to bring about the replication or expression of the segment. The vector may particularly be a plasmid, a cosmid, a virus or a bacteriophage used conventionally in genetic engineering that comprise a polynucleotide encoding tau protein or a variant thereof. Expression vectors derived from viruses such as retroviruses, vaccinia virus, adeno-associated virus, herpes viruses, or bovine papilloma virus, may be used for delivery of the polynucleotide or vector into the cells of the brain slice. Methods which are well known to those skilled in the art can be used to construct recombinant viral vectors; see, for example, the techniques described in Sambrook et al., "Molecular Cloning, A Laboratory Manual, 2nd ed. 1989, CSH Press, Cold Spring Harbor, N.Y. and Ausubel et al., Current Protocols in Molecular Biology, Green Publishing Associates and Wiley Interscience, N.Y. (1989). Alternatively, the polynucleotides and vectors described herein can be reconstituted into liposomes for delivery to target cells. The vectors containing the polynucleotide described herein can be transferred into the host cell by well-known techniques, which vary depending on the type of cellular host. Preferably, the vector is a viral vector, more preferably it is a herpes simplex virus vector or a lentiviral vector. Vectors suitable for transfection of brain slice cultures are described, e.g. in Lilley & Coffin (2003) and Lilley et al. (2001).

The term "recombinant" means, for example, that a polynucleotide sequence is made by an artificial combination of two otherwise separated segments of sequence, e.g., by chemical synthesis or by the manipulation of isolated polynucleotides by genetic engineering techniques

The term "polynucleotide" generally refers to any polyribonucleotide or polydeoxyribonucleotide that may be unmodified RNA or DNA or modified RNA or DNA. The polynucleotide may be single- or double-stranded DNA, single or double-stranded RNA. As used herein, the term "polynucleotide" also includes DNAs or RNAs. It will be appreciated that a variety of modifications may be made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including, for example, simple and complex cells.

The brain slice is usually transfected or transduced by contacting the brain slice with the vector. Preferably, the transfection or transduction is performed in a manner such that pyramidal neurons are transfected or transduced. In addition, it is preferred to minimize vector consumption. For that purpose, a microdroplet is placed roughly onto the CA1 region of each individual slice. The microdroplet has a volume of from about 0.04 µl to about 0.2 µl, preferably of from about 0.05 µl to about 0.15 µl, more preferably of from about 0.06 µl to about 0.1 µl, even more preferably of from about 0.07 µl to about 0.09 µl, most preferably of about 0.08 µl. The microdroplet may be applied using a syringe, e.g. a 1 µl syringe, such as a 1 µl Hamilton syringe. This embodiment is preferably used for viral transduction of hippocampal slice cultures. It may be used, however, also for transfection or transduction of slices from other brain regions, e.g. cortex or midbrain.

### APP

In a specific embodiment, the method further comprises the step of contacting said at least one brain slice with β-amyloid precursor protein (β-APP) or a fragment or derivative or variant thereof. In another embodiment, the method further comprises the step of transfecting or transducing said at least one brain slice with a recombinant vector comprising a polynucleotide encoding β-amyloid precursor protein or a fragment or derivative or variant thereof. The preferred fragment is the β-amyloid peptide Aβ₁₋₄₂. The β-amyloid peptide is derived from a larger Type I membrane spanning protein, β-APP, which has several alternatively spliced transcripts. The amino acid sequence of β-APP and Aβ₁₋₄₂ are described in. Kang J. et al., 1987;: Knauer M.F et al., 1992; Homo sapiens APP (Gen-ID): NM 201414.

The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or, acetylation, or lipidation, or by altered signal peptide cleavage or other types of maturation cleavage. These processes may occur post-translationally.

Another aspect of the present invention is a modified brain slice culture obtainable by a method described hereinabove. In yet another aspect the invention relates to a modified brain slice culture, comprising at least one brain slice which has been transfected or transduced with a recombinant vector which comprises a polynucleotide encoding tau protein or a variant thereof. The preferred embodiments of the modified brain slice culture of the present invention correspond to the preferred embodiments described supra with respect to the method of the invention.

The invention further relates to a test system or cell system which recapitulates tau-linked neurodegeneration, obtainable by a method described hereinabove. The test system or the cell system comprises at least one transfected or transduced brain slice as described herein.

Another aspect of this invention is a method for identifying an agent for treating or preventing neurogenerative disease, comprising using the modified brain slice culture described hereinabove. Yet another aspect of this invention is a method for identifying an agent for treating or preventing neurogenerative disease, comprising (a) contacting a test compound with the modified brain slice culture according to the present invention; and (b) determining whether the test substance modulates at least one marker indicative of the neurogenerative disease.

Neurodegenerative diseases or disorders according to the present invention comprise Alzheimer's disease, Parkinson's disease, Huntington's disease, amyotrophic lateral sclerosis, prion diseases, Pick's disease, fronto-temporal dementia, progressive nuclear palsy, corticobasal degeneration, cerebro-vascular dementia, multiple system atrophy, and mild-cognitive impairment. Further conditions involving neurodegenerative processes are, for instance, ischemic stroke, age-related macular degeneration, narcolepsy, motor neuron diseases, nerve injury and repair, and multiple sclerosis.

Preferably, the neurodegenerative disease is selected from the group consisting of Alzheimer's disease, Parkinson's disease, multiple sclerosis, amyotrophic lateral sclerosis, huntington's disease, tauopathies and prion diseases. Most preferably, the neurodegenerative disease is Alzheimer's disease.

The phrase "marker" as used herein refers to any parameter that indicates the onset or presence of a neurodegenerative disease. Preferred markers include the presence of neurofibrillary tangles, phosporylation of tau, dendritic/axonal dystrophy, axonal degeneration, β-amyloid production and synaptic dystrophy.

The phosporylation of tau which can be determined may be at Ser202, Thr205, S212, S396 or S422 of tau (Kosik & Shimura, 2005). The sites of phosphorylation described in Kosik & Shimura (2005) are incorporated herein by reference. Also Sergeant et al. (2005) Biochimica et Biophysica Acta 1739:179-197 describe sites of phosphorylation which are incorporated herein. The axonal degeneration which may be determined in the assay system is preferably Wallerian degeneration.

A test compound "modulates" a marker indicative of a neurodegenerative disease when it is capable of changing or altering the level and/or the activity of the marker. Said modulation may be an increase or a decrease in the level and/or the activity of the marker. For instance, said modulation may be an increase or decrease in phosporylation of tau, dendritic/axonal dystrophy, axonal degeneration, synaptic dystrophy and/or the amount of neurofibrillary tangles present.
- The extent of phosphorylation of tau at the respective epitopes may be determined using suitable antibodies which specifically recognize phosphoepitopes of tau or non-phosphorylated regions of tau. Such antibodies have been described, for instance, in Johnson & Hartigan (1999) and are commercially available. Modified brain slice of this invention are usually labelled with these antibodies and visualized by indirect immunofluorescence microscopy according to protocols generally known in the art. Quantification of Tau phosphoepitopes can be done by Western blotting followed by densitometrical analysis of the signals or by ELISA according to standard procedures.
- Dendritic or axonal dystrophy/degeneration can be determined by biochemical determination, visual inspection and/or tissue section (staining) labelling employing suitable markers (etc. antibodies detecting the heavy isoform of neurofilament as detailed in Mack et al. 2001B). Morphologically, Wallerian degeneration of axons has been described in detail in Beirowski et al. (2005).
- Synaptic dystrophy can be determined by biochemical determination, visual inspection and/or tissue section labelling (staining) employing suitable markers such as synapsin or synaptophysin (Rutten et al., 2005).
- The formation of neurofibrillary tangles in cells which express a recombinant gene coding for tau protein or an isoform thereof and which has been co-treated with APP, or a fragment, or derivative, or variant thereof, in particular β-amyloid, may be determined by the Gallyas silver impregnation method followed by light/ electron microscopy or other appropriate methods (Braak & Braak, 1995). One such other appropriate method comprises the utilization of conformation-dependent antibodies that are capable of recognizing and discriminating the tau molecule in the context of neurofibrillary tangles from tau molecules existing in other states of aggregation and that can be detected, for instance, by fluorescence microscopy and/or fluorescence resonance energy transfer (FRET) technology. The formation of neurofibrillary tangles could also be detected by the use of other optical methodologies such as fluorescence polarisation spectroscopy, fluorescence correlation spectroscopy, fluorescence cross-correlation spectroscopy, fluorescence intensity distribution analysis, fluorescence lifetime measurements, fluorescence anisotropy measurements, or combinations thereof. For instance, an assay to monitor and quantify the formation and aggregation of paired helical filaments in solution has been described by Friedhoff et al. (1998, Biochemistry, 37: 10223-30). In a preferred embodiment, said conformation-dependent antibodies are optically labeled, preferably flourescently labeled.

In a specific embodiment, the method further comprises the step of co-treatment of the modified brain slice with β-amyloid precursor protein (β-APP) or a fragment or derivative or variant thereof (see supra).

The step of determining whether the test substance modulates at least one marker indicative of the neurodegenerative disease may comprise the following steps: (i) measuring the marker in the modified brain slice culture which has been contacted with the test substance; (ii) measuring the marker in a modified brain slice culture which has not been contacted with the test substance (control brain slice culture); and optionally (iii) comparing the level or activity of marker determined in (i) and (ii).

The method may comprise the step of selecting or identifying as an agent for treating or preventing neurodegenerative disease a test compound which is capable of decreasing the level or activity of the marker indicative of the neurodegenerative disease. The test compound may be selected if it is capable of significantly decreasing the level or activity of the marker. Preferably, if the marker can be measured in a quantitative manner, the test compound is selected if it is capable of decreasing the level or activity of the marker by at least 10 %, preferably by at least 20%, more preferably by at least 30%, even more preferably by at least 40%, most preferably by at least 50% as compared to the control brain slice culture.

The method may further comprise the step of measuring the viability of the modified brain slice culture, in particular of the neurons in the slice culture. This may be done by using methods such as: visual inspection under a microscope; staining using vital dyes stains and immunohistochemical reagents specific for cell types or moieties present in normal and injured brain; reaction with antibodies to neurofilaments, glial fibrillary acidic protein, S100, microtubule associated proteins, and synaptic proteins; biochemical assessment of metabolic activity; measurement of total or specific protein content; assessment of cellular function; and assessment of neural activity.

The viability/integrity of the organotypic slice culture may be assessed at the initiation of each experiment in order to demonstrate the health of the preparation as well as to provide a measure of the amount of viable tissue present in the pretreated culture.

### Test Compound

Compounds that are assayed in the above method can be randomly selected or rationally selected or designed. As used herein, a compound is said to be randomly selected when the compound is chosen randomly without considering the structure of other identified active compounds. An example of randomly selected compounds is the use a chemical library, a peptide combinatorial library, a growth broth of an organism, or a plant extract.

As used herein, a compound is said to be rationally selected or designed when the compound is chosen on a nonrandom basis. Rational selection can be based on the target of action or the structure of previously identified active compounds. Specifically, compounds can be rationally selected or rationally designed by utilizing the structure of compounds that are presently being investigate for use in treating Alzheimer's disease.

The test compounds can be, as examples, peptides, small molecules, and vitamin derivatives, as well as carbohydrates. The test compounds may be nucleic acids, natural or synthetic peptides or protein complexes, or fusion proteins. They may also be antibodies, organic or inorganic molecules or compositions, drugs and any combinations of any of said agents above. They may be used for testing, for diagnostic or for therapeutic purposes. A skilled artisan can readily recognize that there is no limit as to the structural nature of the test compounds to be used in accordance with the present invention.

### Application of Test Substance

At the commencement of an experiment, an organotypic slice culture is typically transferred to a culture dish with media. The culture media can either have a test compound present prior to the introduction of the tissue section or a test compound can be added to the media after the tissue section has been placed in the culture dish. In general, a test substance will be first dissolved in appropriate vehicle, such as, but not limited to, DMSO, water, physiological saline, or media, to make a stock solution and then diluted into the media. A vehicle control test may be included when the present invention is used.

Preferably, a range of doses is tested. The range tested initially may be informed by prior knowledge of the effects of the test compound or closely related substances on purified proteins, cells in culture, or toxicity in other test systems. In the absence of such knowledge, the dose range is preferably from about 1 nM to about 100 µM. A skilled artisan can readily develop a testing range for any particular compound or series of compounds.

The test compound is typically applied to the modified brain slice for about 4 hours to about 21 days, preferably from about 1 day to about 7 days. In the case of long term application, fresh media containing test compound can be applied periodically; more frequently if rapid loss of test compound due to chemical conversion or to metabolism is suspected.

Another aspect of the present invention is the use of a modified slice culture according to the present invention for screening an agent or agents capable of modulating one or more markers of neurodegenerative disease.

In another aspect, the invention relates to the use of a modified slice culture according to the present invention for the development of medicaments for the treatment or prevention of neurodegenerative diseases.

In yet another aspect, the invention pertains to the use of a modified slice culture according to the present invention for determining the toxicity of test compounds. Toxicity testing may be performed by using propidium iodide as a marker selectively penetrating injured cells but excluded from healthy cells. Alternatively, Fluoro-Jade B may be used as a marker labelling spezifically degenerating neurons but no other cell types, which has been successfully tested in an *in vivo* tauopathy mouse model.

It has been found in the present invention that ex vivo model expression of tau_{P301L} in pyramidal neurons causes age-dependant degeneration of dendrites as well as axons. Thus, in addition to known markers of etiopathology, this *ex vivo* model provides previously unattainable details to progressive etiopathology, enabling a better tool for screening agents capable of modulating ethiopathology in a cell.

Figure 1: Scheme of high throughput ex vivo compound screening.
*Ex vivo* models combine disease-relevant, complex pathomechanisms with handling and throughput characteristics of cell-based *in vitro* models. Moreover, they approximate significance and predictive value of animal models.

Figure 2: Expression of human Tau_{P301L} in hippocampal culures.
Hippocampal slice cultures were incubated for 5 days under serum free conditions and infected with high titre Herpes virus vectors transducing a human tau 0N4R construct (harbouring the P301 L mutation). Accumulating levels of human tau_{P301} were detected in pyramidal neurons in the CA neuronal band of the slices using antibodies labelling all isoforms of human tau but not endogenous rat tau. Tau_{P301} protein was first evident in processes (i.e. dendrites and axons) of pyramidal neurons between 1 and 2 days after transduction (A and B) and continuously accumulated during the following days in culture (C and D). The highest density of transduced neurons in the slices were observed in the CA3 region, thus representative images using constant camera parameters were taken from this region. Scale bars = 50 µM.

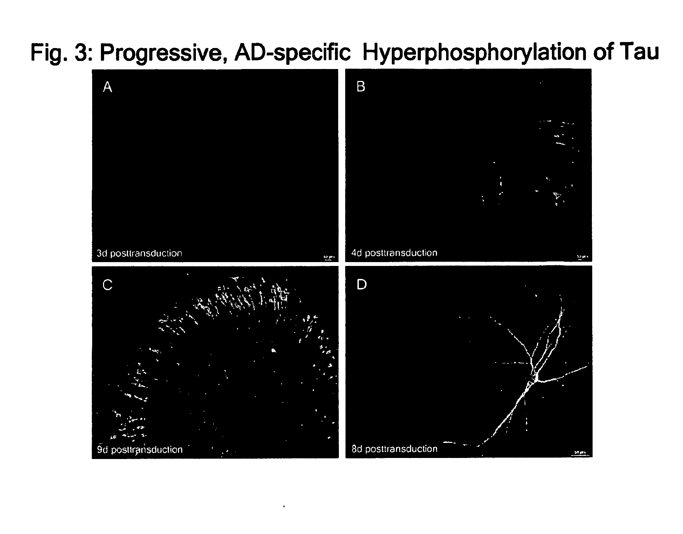

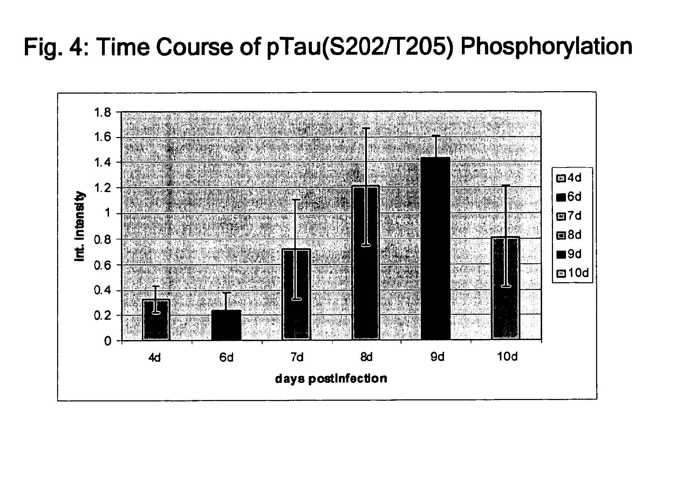

Figure 3: Progressive, AD-specific hyperphosphorylation of Tau.
Transgenic but not endogenous tau in transduced slices was shown to be abnormally phosphorylated at Serine 202 and Threonine 205 labelled by the AT8 antibody as an early marker preceding neurodegeneration in Alzheimer brains. Pathological phosphorylation started as early as 4 days posttransduction and progressed up to 9 days posttransduction in pyramidal neurons of the CA3 region (A - C). Moreover, the AT8 epitope represents a diagnostic marker in that it anticipates axon or dendrite degeneration. In contrast to neurons shown in Fig. 4 neither dendrite nor axon degeneration has started in individual neurons although pathological AT8 phosphorylation was already prominent (D). Within the same slice, neurons passing through various stages of dendrite/axon degeneration or even before the start of degenerating events could be found in close proximity, similarly labelled by the AT8 marker (not shown). Scale bars = 50 µM.

Figure 4: Time Course of pTau (S202/T205) Phosphorylation.
In order to quantify pathological phophorylation at the diagnostic AT8 epitope image analysis was used to determine the time course of hyperphosphorylation. A progressive age-dependent increase of Alzheimer-relevant AT8 phosphorylation led to peak levels between 8 and 9 days after which levels may drop due to progressive neurodegeneration.

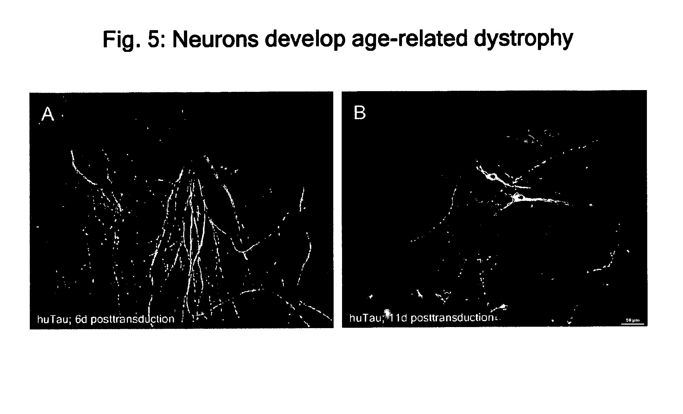

Figure 5: Neurons develop age-related dystrophy.
Expression of human tau_{P301} in pyramidal neurons, labelled by an antibody detecting all human tau isoforms, caused age-dependant degeneration of dendrites as well as axons responsible for functional communication between hippocampal neurons. Dendritic dystrophy became evident as partly fragmented processes displaying evenly spaced swellings (A). Interestingly, dystrophic processes in neurons were evident in individual neurons before tau accumulated in the cell body, i.e. presumably before NFT formation in the cell body may start. Gallyas silver impregnation would be needed to analyse whether insoluble tau aggregates in the form of neuropil threads are present in these dystrophic dendrites. Following dendritic degeneration, axon degeneration was observed (B). Processes most likely representing axons from CA3 pyramidal neurones turning into the Schaffer Collateral pathway connecting with CA1 neurones show clear morphological characteristics of Wallerian degeneration, i.e. axonal bead-like swellings and progressive fragmentation. Scale bars = 50 µM.

The following non-limiting examples further illustrate the invention. It should be understood, however, that the invention as claimed should not be unduly limited to such specific embodiments. Indeed, various modifications of the described modes for carrying out the invention which are obvious to those skilled in the art are intended to be within the scope of the claims.

### Examples

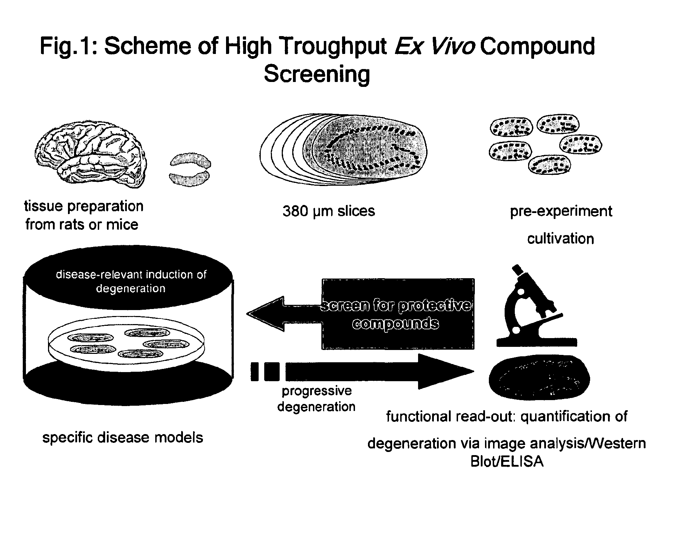

The aim of this method is to recapitulate aspects of the complex Alzheimer disease pathogenesis in a tissue-based ex *vivo* model in order to provide a tool for rapid and significant compound screening during pre-clinical drug development. The general principle of tissue-based functional screening is summarized in Fig. 1. Disease-relevant tissue is prepared from healthy animals and manipulated *ex vivo* to develop progressive signs of disease progression. Tissue-based models are in particular relevant if complex disease pathomechanisms rely on cell communication involving different cell types in a three dimensional network. Disease-relevant functional parameters can be monitored with a much higher throughput compared to animal models and compounds can be applied as easily as in a cell culture model. *Ex vivo* models therefore combine accessibility and throughput of *in vitro* models with complexity, significance and predictive value of animal models.

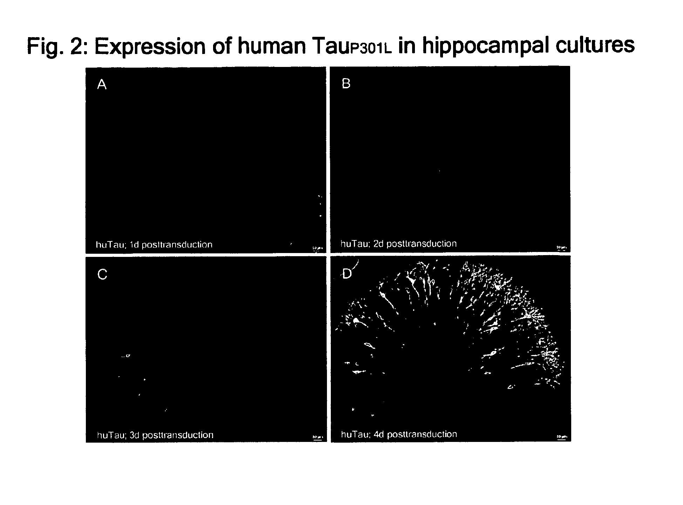

In order to transduce hippocampal neurons ex *vivo* with a human tau variant (P301L) slices cultures were incubated for 5 days under serum free conditions and infected with high titre Herpes virus vectors. Transgene expression was surprisingly distinct in pyramidal neurons and could be in detail monitored with antibodies detecting all isoforms of human tau but not endogenous rat tau (Fig.2; images were taken with constant exposure parameters to appreciate increasing expression levels). Rising levels of human tau_{P301} were evident in pyramidal neurons in the CA neuronal band of the slices. Tau_{P301} protein was first detected in processes (i.e. dendrites and axons) of pyramidal neurons between 1 and 2 days after transduction and continuously accumulated during the following days in culture. Progressive tau_{P301} protein expression led to increasing numbers of neurons that in addition to labelled processes showed accumulation of the transgene in the cell bodies (Fig. 2d). The highest density of transduced neurons in the slices were observed in the bent CA3 region, thus representative images were taken from this region to exclude regional differences in expression levels.

To investigate neuropathological changes we analysed transduced slices with a diagnostic marker specific for abnormal hyperphosphorylated tau that precedes and predicts NFT formation in Alzheimer-affected brains (Braak & Braak, 1995; Fig. 3). Transgenic tau was shown to be abnormally phosphorylated at Serine 202 and Threonine 205 known as the AT8 epitope. Pathological phosphorylation started as early as 4 days posttransduction and progressed up to 9 days posttransduction (Fig. 3b, c). Thus, we conclude that high tau_{P301L} expression levels reached 4 days posttransduction were sufficient to induce age-related abnormal accumulation of protein concomittant with pathological phosphorylation indicative of Alzheimer-specific alterations in neuron metabolism. Moreover, we detected neurons with full blown AT8-hyperphosphorylation (Fig. 3d) but no apparent signs of neurodegeneration as compared to neurons shown in Fig. 5. This observation suggests that AT8-type phosphorylation precedes neurodegeneration in our model system as an early marker for Alzheimer-specific pathological changes similar to the situation in Alzheimer patients (Braak & Braak, 1995). Thus, the diagnostic marker AT8 anticipates degeneration and, in contrast to animal models, provides in our model system a quantifiable parameter for drug testing (s. also Fig. 4). In animal models, NFT pathology has been used as quantitative readout although recent data have questioned the correlation between NFT pathology and cognitive decline or neuronal death (SantaCruz et al., 2005). Therefore, given that diagnostic abnormal phosphorylation precedes and anticipates neurodegeneration that does correlate with cognitive decline and behavioural alterations our ex vivo model may partly replace time consuming and expensive *in vivo* testing providing valid data on compound efficacy with focus on combating pathological tau phosphorylation.

Different levels of tau_{P301} expression were detected in individual neurons within one slice but also variations of expression levels between individual slices within one experiment. In order to quantify pathological phophorylation at the diagnostic AT8 epitope image analysis was used to determine the time course of hyperphosphorylation (Fig. 4). We detected an age-dependent increase of Alzheimer-relevant tau phosphorylation after 7 days posttransduction. Peak levels were evident between 8 and 9 days after which levels may drop due to progressive neurodegeneration.

In addition, the full range of tau phosphoepitopes indicative of Alzheimer-specific abnormal tau hyperphosphorylation may be used as markers in this model. While the specific neuronal tau kinase/kinases responsible for phosphorylation of tau at the AT8 epitope are still unknown, other kinases have been attributed towards certain other phosphoepitopes. Since recent results support the idea that kinases are involved in tauopathy progression (Noble et al., 2005) there seems to be a need for relevant ex *vivo* models to test specific tau kinase inhibitors in hippocampal tissue. Thus, quantification of AD-specific phosphoepitopes will provide a by far more pathology-relevant quantitative tool for screening of therapeutic kinase inhibitors as compared to conventional kinase assays.

Several epitopes that have been found disease-specific were not detectable in immunofluorescence but readout relied on Western blotting and/or ELISA. For example, using pS396 as a marker epitope we were able to detect a specific signal 9 days postinfection (not shown). Since this epitope has been attributed to glycogen synthase kinase 3 β, inhibitors of this kinase can now be tested in this model and preliminary attempts are under way. Other relevant epitopes include pS422 and pS212 (also known as the AT100 epitope, respectively). In addition, conformational epitopes like MC1 may prove diagnostic also in this model. Moreover, markers specific for neurodegneration as Fluoro-Jade B may prove useful as concomitant markers of disease-relevant metabolic changes.

Synaptic and dendrite/axon dystrophies are regarded as very early disease symptoms cutting off connectivity between neurons. This progressive degeneration impairs hippocampal circuits underlying memory and cognitive functions long before neuronal cell death. Axon degeneration is considered as an early sign of degeneration in the spinal cord of animal models of tauopathy but, to our knowledge, dendritic and axon degeneration have not been as pronounced in Alzheimer-relevant brain regions in animal models.

Surprisingly, in our *ex vivo* model expression of tau_{P301} in pyramidal neurons caused age-dependant degeneration of dendrites as well as axons responsible for functional communication between cells. Dendritic dystrophy became evident as partly fragmented processes displaying evenly spaced swellings (Fig. 5a). Interestingly, dystrophic processes in neurons were detectable before tau accumulated in the cell body. Following dendritic dystrophy axon degeneration was observed (Fig. 5b). Processes most likely representing axons from CA3 pyramidal neurones turning into the Schaffer Collateral pathway connecting with CA1 neurones show clear signs of Wallerian degeneration, i.e. axonal swellings and progressive fragmentation. Thus, in addition to known markers of pathology our model provides previously unknown details to progressive etiopathology. Given that dendritic and axonal dystrophy in the hippocampus are very early symptoms of disease-relevant cognitive impairment and memory dysfunction, the integrity of these structures seems an attractive target of a successful protective anti-degenerative strategy.

In summary, our novel ex *vivo* model is anticipated to provide direct evidence of protective potency of drugable compounds focussing on preservation of neuron integrity and connectivity. Most importantly, our tissue-based AD model can serve as a cost-effective means to develop a rational design of therapeutic strategies to treat AD and related tauopathies.

### Methods:

### Organotypic cultures

Hippocampal slice cultures were prepared from 8-9 days old rats as has been described (Noraberg et al., 2005; and references therein). A novel method was established in order to increase pyramidal neuron infection efficacy and minimise viral vector consumption at the same time, thus making the assay suitable for higher throughput screening. A micro droplet of approximately 0.08 µl is placed roughly onto the CA1 region of each individual slice using a 1 µl Hamilton syringe (i.e. the dorso-central region of the slice). All experiments were done in triplicate (i.e. treatment of 3 membranes from different animals harbouring between 5-6 slices each) and repeated at least once in an independent set of experiments. Although the method for viral transduction was optimised for hippocampal slice cultures, it may be applied to other regions of the brain including cortex, midbrain etc.

Control experiments employing eGFP-expressing viral vectors driven by the same promoter confirm prominent reproducible transduction of pyramidal neurons in the CA3 and, to a somewhat lesser extent, in the CA1 region of the hippocampal slice (not shown). No apparent toxicity during the observation period was observed with either propidium iodide as marker for cell death nor decrease or dystrophy of GFP-expressing neurons. Suitable viral vectors comprise Herpes simplex type 1 (HSV-1) derived vectors (Biovex; Oxford, UK), lentiviral vectors (ViraPower Lentiviral Expression System, Invitrogen) and potentially others that have not been tested yet.

### Viral vectors

Construction of multiply disabled herpes simplex viral vectors for gene delivery to the nervous system has been described (Lilley & Coffin, 2003) and, in particular, construction of a GFP-expressing viral vector capable of infecting postmitotic neurons *ex vivo* was detailed previously (Lilley et al., 2001). Mutant variants of human tau protein have been found to cause familial forms of FTDP-17 (Hutton et al., 1988). The 0N4R isoform of human tau harbouring the P301L mutation was chosen because it most rapidly accumulated in differentiated neuronal cells amongst all analysed mutant isoforms (Mack et al., 2001A) but other mutations may lead to similar if not identical pathomechanisms. Cloning was done by high fidelity PCR using primers that allow for directional TOPO cloning 3' to a CMV promoter driving high levels of expression. Alternatively, the 3' part of the CaMKII promoter was used to ensure expression selectively in hippocampal pyramidal neurons (not shown). Virus production was done according to the manufacturers instructions (Invitrogen).

### Immunohistochemistry and Image analysis

Tau isoforms were labelled by indirect immunoflurescence as has been described (Mack et al., 2001A) with minor modifications. Slices were fixed in 4% PFA for 1h followed by permeabilization over night with 0.5 % Triton X-100 in PBS. The following antibodies were used for labelling: Tau-2 (Sigma-Aldrich; 1:1000); Tau-13 (Covance; 1:5000), AT8 (Innogenetics; 1:250) and Alexa 594 goat anti-mouse (Molecular probes; 1:250).
Images were acquired on a Nikon inverse microscope equipped with a CCD camera using LUCIA software (Laboratory Imaging; Prague; Czech Republic). All images used for quantification were captured employing constant camera parameters and exposure time (200 ms). Integrated intensity was calculated from these images using an empirically validated threshold between signal and background fluorescence in order to analyse the frequency of labelled cells as well as their intensity.

### References

Beirowski, B., Adalbert, R., Wagner, D., Grumme, D. S., Addicks, K., Ribchester, R. R. and Coleman, M. P. (2005). The progressive nature of Wallerian degeneration in wild-type and slow Wallerian degeneration (WldS) nerves. BMC Neurosci 6, 6.
Braak, H. and Braak, E. (1995). Staging of Alzheimer's disease-related neurofibrillary changes. Neurobiol Aging 16, 271-8; discussion 278-84.
Ferrari, A., Hoerndli, F., Baechi, T., Nitsch, R. M. and Gotz, J. (2003). beta-Amyloid induces paired helical filament-like tau filaments in tissue culture. J Biol Chem 278, 40162-8.
Friedhoff, P., Schneider, A., Mandelkow, E. M. and Mandelkow, E. (1998). Rapid assembly of Alzheimer-like paired helical filaments from microtubule-associated protein tau monitored by fluorescence in solution. Biochemistry 37, 10223-30.
Hardy, J. and Selkoe, D. J. (2002). The amyloid hypothesis of Alzheimer's disease: progress and problems on the road to therapeutics. Science 297, 353-6.
Higgins, G. A. and Jacobsen, H. (2003). Transgenic mouse models of Alzheimer's disease: phenotype and application. Behav Pharmacol 14, 419-38.
Higgins, L. S., Rodems, J. M., Catalano, R., Quon, D. and Cordell, B. (1995). Early Alzheimer disease-like histopathology increases in frequency with age in mice transgenic for beta-APP751. Proc Natl Acad Sci U S A 92, 4402-6.
Hutton, M. (2001). Missense and splice site mutations in tau associated with FTDP-17: multiple pathogenic mechanisms. Neurology 56, S21-5.
Hutton, M., Lendon, C. L., Rizzu, P., Baker, M., Froelich, S., Houlden, H., Pickering-Brown, S., Chakraverty, S., Isaacs, A., Grover, A. et al. (1998). Association of missense and 5'-splice-site mutations in tau with the inherited dementia FTDP-17. Nature 393, 702-5.
Johnson, G. V. and Hartigan, J. A. (1999). Tau protein in normal and Alzheimer's disease brain: an update. J Alzheimers Dis 1, 329-51.
Kang, J., Lemaire, H. G., Unterbeck, A., Salbaum, J. M., Masters, C. L., Grzeschik, K. H., Multhaup, G., Beyreuther, K. and Muller-Hill, B. (1987). The precursor of Alzheimer's disease amyloid A4 protein resembles a cell-surface receptor. Nature 325, 733-6.
Knauer, M. F., Soreghan, B., Burdick, D., Kosmoski, J. and Glabe, C. G. (1992). Intracellular accumulation and resistance to degradation of the Alzheimer amyloid A4/beta protein. Proc Natl Acad Sci U S A 89, 7437-41.
Ko, L. W., Rush, T., Sahara, N., Kersh, J. S., Easson, C., Deture, M., Lin, W. L., Connor, Y. D. and Yen, S. H. (2004). Assembly of filamentous tau aggregates in human neuronal cells. J Alzheimers Dis 6, 605-22; discussion 673-81.
Kosik, K. S. and Shimura, H. (2005). Phosphorylated tau and the neurodegenerative foldopathies. Biochim Biophys Acta 1739, 298-310.
LaFerla, F. M. and Oddo, S. (2005). Alzheimer's disease: Abeta, tau and synaptic dysfunction. Trends Mol Med 11, 170-6.
Lewis, J., Dickson, D. W., Lin, W. L., Chisholm, L., Corral, A., Jones, G., Yen, S. H., Sahara, N., Skipper, L., Yager, D. et al. (2001). Enhanced neurofibrillary degeneration in transgenic mice expressing mutant tau and APP. Science 293, 1487-91.
Lewis, J., McGowan, E., Rockwood, J., Melrose, H., Nacharaju, P., Van Slegtenhorst, M., Gwinn-Hardy, K., Paul Murphy, M., Baker, M., Yu, X. et al. (2000). Neurofibrillary tangles, amyotrophy and progressive motor disturbance in mice expressing mutant (P301 L) tau protein. Nat Genet 25, 402-5.
Lilley, C. E. and Coffin, R. S. (2003). Construction of multiply disabled herpes simplex viral vectors for gene delivery to the nervous system. Methods Mol Med 76, 33-49.
Lilley, C. E., Groutsi, F., Han, Z., Palmer, J. A., Anderson, P. N., Latchman, D. S. and Coffin, R. S. (2001). Multiple immediate-early gene-deficient herpes simplex virus vectors allowing efficient gene delivery to neurons in culture and widespread gene delivery to the central nervous system in vivo. J Virol 75, 4343-56.
Mack, T. G., Dayanandan, R., Van Slegtenhorst, M., Whone, A., Hutton, M., Lovestone, S. and Anderton, B. H. (2001A). Tau proteins with frontotemporal dementia-17 mutations have both altered expression levels and phosphorylation profiles in differentiated neuroblastoma cells. Neuroscience 108, 701-12.
Mack, T. G., Reiner, M., Beirowski, B., Mi, W., Emanuelli, M., Wagner, D., Thomson, D., Gillingwater, T., Court, F., Conforti, L. et al. (2001 B). Wallerian degeneration of injured axons and synapses is delayed by a Ube4b/Nmnat chimeric gene. Nat Neurosci 4, 1199-206.
Mandelkow, E. M. and Mandelkow, E. (1998). Tau in Alzheimer's disease. Trends Cell Biol 8, 425-7.
Noble, W., Planel, E., Zehr, C., Olm, V., Meyerson, J., Suleman, F., Gaynor, K., Wang, L., LaFrancois, J., Feinstein, B. et al. (2005). Inhibition of glycogen synthase kinase-3 by lithium correlates with reduced tauopathy and degeneration in vivo. Proc Natl Acad Sci U S A 102, 6990-5.
Noraberg, J., Poulsen, F. R., Blaabjerg, M., Kristensen, B. W., Bonde, C., Montero, M., Meyer, M., Gramsbergen, J. B. and Zimmer, J. (2005). Organotypic hippocampal slice cultures for studies of brain damage, neuroprotection and neurorepair. Curr Drug Targets CNS Neurol Disord 4, 435-52.
Oddo, S., Caccamo, A., Shepherd, J. D., Murphy, M. P., Golde, T. E., Kayed, R., Metherate, R., Mattson, M. P., Akbari, Y. and LaFerla, F. M. (2003). Triple-transgenic model of Alzheimer's disease with plaques and tangles: intracellular Abeta and synaptic dysfunction. Neuron 39, 409-21.
Quon, D., Wang, Y., Catalano, R., Scardina, J. M., Murakami, K. and Cordell, B. (1991). Formation of beta-amyloid protein deposits in brains of transgenic mice. Nature 352, 239-41.
Rutten, B. P., Van der Kolk, N. M., Schafer, S., van Zandvoort, M. A., Bayer, T. A., Steinbusch, H. W. and Schmitz, C. (2005). Age-related loss of synaptophysin immunoreactive presynaptic boutons within the hippocampus of APP751SL, PS1M146L, and APP751SL/PS1M146L transgenic mice. Am J Pathol 167, 161-73.
Santacruz, K., Lewis, J., Spires, T., Paulson, J., Kotilinek, L., Ingelsson, M., Guimaraes, A., DeTure, M., Ramsden, M., McGowan, E. et al. (2005). Tau suppression in a neurodegenerative mouse model improves memory function. Science 309, 476-81.
Sergeant, N., Delacourte, A. and Buee, L. (2005). Tau protein as a differential biomarker of tauopathies. Biochim Biophys Acta 1739, 179-97.

## Claims

1. A method for preparing a modified brain slice culture, comprising transfecting or transducing at least one brain slice with a recombinant vector which comprises a polynucleotide encoding tau protein or an isoform thereof.

2. A method according to claim 1, wherein the brain slice is a hippocampal slice.

3. A method according to claim 1 or 2, wherein the brain slice is derived from rat brain or mouse brain.

4. A method according to any one of the preceding claims, wherein the vector is a viral vector.

5. A method according to any one of the preceding claims, wherein the tau isoform is 0N4R huTau P301 L.

6. A method according to any one of the preceding claims, wherein the transfection or transduction is transient.

7. A method according to any one of the preceding claims, further comprising the step of contacting said at least one brain slice with β-amyloid precursor protein or a fragment or derivative or variant thereof.

8. A method according to any one of claims 1 to 6, further comprising the step of transfecting or transducing said at least one brain slice with a recombinant vector comprising a polynucleotide encoding β-amyloid precursor protein or a fragment or derivative or variant thereof.

9. A method according to claim 7 or 8, wherein the fragment is Aβ₁₋₄₂.

10. A modified brain slice culture obtainable by a method according to any one of claims 1 to 9.

11. A modified brain slice culture, comprising at least one brain slice which has been transfected or transduced with a recombinant vector which comprises a polynucleotide encoding tau protein or an isoform thereof.

12. A method for identifying an agent for treating or preventing neurogenerative disease, comprising using the modified brain slice culture according to claim 10 or 11.

13. A method for identifying an agent for treating or preventing neurogenerative disease, comprising
(a) contacting a test compound with the modified brain slice culture according to claim 10 or 11;
(b) determining whether the test substance modulates at least one marker indicative of the neurogenerative disease.

14. A method according to claim 13, wherein the neurodegenerative disease is selected from the group consisting of Alzheimer's disease, Parkinson's disease, multiple sclerosis, amyotrophic lateral sclerosis, huntington's disease, tauopathies and prion diseases.

15. A method according to claim 13 or 14, wherein the marker indicative of the neurodegenerative disease is selected from the group consisting of neurofibrillary tangles, phosporylation of tau, dendritic/axonal dystrophy, axonal degeneration, and synaptic dystrophy.

16. A method according to claim 15, wherein the phosporylation of tau is at Ser202, Thr205, S212, S396 or S422 of tau.

17. A method according to claim 15, wherein the axonal/dendritic degeneration (includes) shares morphological and/or metabolical characteristics with Wallerian degeneration.

18. The use of a modified slice culture according to claim 10 or 11 for screening an agent or agents capable of modulating one or more markers of neurodegenerative disease.

19. The use of a modified slice culture according to claim 10 or 11 for the development of medicaments for the treatment or prevention of neurodegenerative diseases.

20. The use of a modified slice culture according to claim 10 or 11 for determining the toxicity of test compounds.


SEQUENCE LISTING

<110>  KeyNeurotek AG

<120>  A tissue-based assay system for Alzheimer-specific Degeneration
       and Pathology

<130>  KeyNeurotek

<160>  3

<170>  PatentIn version 3.2

<210>  1
<211>  383
<212>  PRT
<213>  homo sapiens

<400>  1

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Ala Glu Glu Ala
        35                  40                  45

Gly Ile Gly Asp Thr Pro Ser Leu Glu Asp Glu Ala Ala Gly His Val
    50                  55                  60

Thr Gln Ala Arg Met Val Ser Lys Ser Lys Asp Gly Thr Gly Ser Asp
65                  70                  75                  80

Asp Lys Lys Ala Lys Gly Ala Asp Gly Lys Thr Lys Ile Ala Thr Pro
                85                  90                  95

Arg Gly Ala Ala Pro Pro Gly Gln Lys Gly Gln Ala Asn Ala Thr Arg
            100                 105                 110

Ile Pro Ala Lys Thr Pro Pro Ala Pro Lys Thr Pro Pro Ser Ser Gly
        115                 120                 125

Glu Pro Pro Lys Ser Gly Asp Arg Ser Gly Tyr Ser Ser Pro Gly Ser
    130                 135                 140

Pro Gly Thr Pro Gly Ser Arg Ser Arg Thr Pro Ser Leu Pro Thr Pro
145                 150                 155                 160

Pro Thr Arg Glu Pro Lys Lys Val Ala Val Val Arg Thr Pro Pro Lys
                165                 170                 175

Ser Pro Ser Ser Ala Lys Ser Arg Leu Gln Thr Ala Pro Val Pro Met
            180                 185                 190

Pro Asp Leu Lys Asn Val Lys Ser Lys Ile Gly Ser Thr Glu Asn Leu
        195                 200                 205

Lys His Gln Pro Gly Gly Gly Lys Val Gln Ile Ile Asn Lys Lys Leu
    210                 215                 220

Asp Leu Ser Asn Val Gln Ser Lys Cys Gly Ser Lys Asp Asn Ile Lys
225                 230                 235                 240

His Val Pro Gly Gly Gly Ser Val Gln Ile Val Tyr Lys Pro Val Asp
                245                 250                 255

Leu Ser Lys Val Thr Ser Lys Cys Gly Ser Leu Gly Asn Ile His His
            260                 265                 270

Lys Pro Gly Gly Gly Gln Val Glu Val Lys Ser Glu Lys Leu Asp Phe
        275                 280                 285

Lys Asp Arg Val Gln Ser Lys Ile Gly Ser Leu Asp Asn Ile Thr His
    290                 295                 300

Val Pro Gly Gly Gly Asn Lys Lys Ile Glu Thr His Lys Leu Thr Phe
305                 310                 315                 320

Arg Glu Asn Ala Lys Ala Lys Thr Asp His Gly Ala Glu Ile Val Tyr
                325                 330                 335

Lys Ser Pro Val Val Ser Gly Asp Thr Ser Pro Arg His Leu Ser Asn
            340                 345                 350

Val Ser Ser Thr Gly Ser Ile Asp Met Val Asp Ser Pro Gln Leu Ala
        355                 360                 365

Thr Leu Ala Asp Glu Val Ser Ala Ser Leu Ala Lys Gln Gly Leu
    370                 375                 380

<210>  2
<211>  441
<212>  PRT
<213>  homo sapiens

<400>  2

<210>  3
<211>  31
<212>  PRT
<213>  homo sapiens

<400>  3

Val Gln Ile Ile Asn Lys Lys Leu Asp Leu Ser Asn Val Gln Ser Lys
1               5                   10                  15

Cys Gly Ser Lys Asp Asn Ile Lys His Val Leu Gly Gly Gly Ser
            20                  25                  30